# EUROPEAN PATENT APPLICATION

(11) **EP 1 484 348 A2**
(43) Date of publication of application: **08.12.2004**
(21) Application number: 04012034.7
(22) Date of filing: 21.05.2004
(51) Int. Cl.: C08G 18/12, C08G 18/66, A61B 19/04

(54) **Modified polyurethanes**

(30) Priority: 03.06.2003 US 453755
(71) Applicant: Bayer MaterialScience LLC, Pittsburgh, PA 15205-9741 (US); Bayer Aktiengesellschaft, 51368 Leverkusen (DE)
(72) Inventor: Gindin, Lyubov K., Pittsburgh, PA 15228 (US); Roesler, Richard R., Wexford, PA 15090-7583 (US); Yu, Poli C., Wexford, PA 15090 (US); Kleer, Joseph R., Crescent, PA 15046-4909 (US); Muenzmay, Thomas, 41539 Dormagen (DE); Berezkin, Yuliya, Pittsburgh, PA 15228-2461 (US); Crisci, Mary A., Aliquippa, PA 15001-5053 (US)
(74) Representative: Perchenek, Nils, Dr.

(57) **Abstract**

The present invention relates to isocyanate functional prepolymers, aqueous polyurethane dispersions produced from the prepolymers and various uses of such dispersions. The prepolymers are prepared by reacting a diisocyanate, a dihydroxy compound having a number average molecular weight of from about 700 to about 16,000, and a trihydroxy component of the formula:

R-(OH)₃

where R is a saturated straight chain or branched chain aliphatic group of from 2 to 8 carbon atoms. The invention also relates to polyurethane dispersions prepared from the prepolymers and to various uses of the resultant dispersions.

## Description

### BACKGROUND OF THE INVENTION

The invention relates to isocyanate-functional polyurethane prepolymers, aqueous dispersions produced from the prepolymers and to the use of the dispersions for producing of materials that can be used in medical applications.

In the early 1990's, general prophylactic measures adopted worldwide against HIV, hepatitis B and hepatitis C led to dramatically increased use of latex-based gloves and condoms. However, significant numbers of personnel working in the health field showed allergic reactions. Natural latex contains type I and type IV allergens. Type I allergens are attributable to the proteins which naturally occur in latex, and can even result in anaphylactic shock. Type IV allergens are the accelerators and additives needed in latex production. These frequently lead to hypersensitive contact dermatitis. These allergic reactions described are not known to result from polyurethanes.

Typical of the many references that relate to the use of polyurethane dispersions for such medical applications are: U.S. patents 5,576,382, 5,985,955, 5,997,969, 6,389,602, 6,440,498, 6,451,908, 6,451,963, and 6,514,572; and published U.S. patent applications 2001/0053815, 2002/0028875 and 2002/0028877.

However, certain problems have arisen with respect to gloves made from polyurethane dispersions. Such gloves are rendered useless when contacted with a sterilizing solution, i.e., alcohol. For example, before a surgeon puts on the gloves, their hands are treated with a Sterillium® solution (disinfectant in isopropanol solvent). Surgical items or tools may also be treated with the disinfectant. The surgeons, however, do not flash off the disinfectant solvent before donning the gloves. As the glove contacts the Sterilium solution, the gloves "break down" by developing holes, cracks and a stickiness to the glove. As a result, the integrity of the glove in protecting the hands of the surgeon as well as the dexterity of the surgeon is compromised.

Canadian patent 1,089,141 describes mixtures of aromatic and aliphatic or cycloaliphatic polyisocyanates for the preparation of finely divided, stable aqueous dispersions of anionically modified polyurethanes. However, the resistance to isopropanol of the flat materials that can be produced from them is poor U.S. patent 6,084,051 describes polyurethane dispersions having improved storage stability. The dispersions described therein are produced from a polyurethane prepolymer that includes trimethylolpropane during its production. However, the overall properties of flat materials that could be produced from them are not satisfactory for medical use.

In addition to isopropanol resistance, in the case of surgical gloves, the gloves must be appropriately flexible and soft. A surgeon may perform surgical procedures for an extended period of time and requires maximum comfort, tactility and grip of the gloves. Additionally, shear stability of the polyurethane dispersion is also a very desirable property since the glove manufacturing process may require the continuous stirring of the dispersion for an extended period of time. During prolonged agitation, a dispersion that retains its initial viscosity, mean particle size and particle size distribution is considered as shear stable. Finally, shelf stability of the dispersion is also important.

An object of the present invention was to provide isocyanate-functional prepolymers suitable for the production of polyurethane flat materials that combine satisfactory solvent resistance with simultaneously good minimum tear strength and minimum ultimate elongation, and which do not have the disadvantages described in the prior art.

### DESCRIPTION OF THE INVENTION

The above-noted object may be achieved by incorporating small quantities of relatively low molecular weight triols in the production of the isocyanate preploymer used to produce into the polyurethane dispersion. The presence of this triol surprisingly gives rise to a polyurethane dispersion with greatly improved isopropanol resistance.

More particularly, the present invention relates to isocyanate functional prepolymers, aqueous polyurethane dispersions produced from the prepolymers and various uses of such dispersions.

The prepolymers of the present invention have an NCO content of from about 1 to about 6% by weight, and are prepared by reacting:
A) an organic diisocyanate,
B) at least one dihydroxy compound having a number average molecular weight of from about 700 to about 16,000, and
C) a trihydroxy component of the formula:

   R-(OH)₃

   where R is a saturated straight chain or branched chain aliphatic group of from 2 to 8 carbon atoms, and
   wherein the amount of component C) is such that the hydroxy groups from component C) amount to from about 2 to about 15 % based on the total amount of hydroxy equivalents used to produce the prepolymer. The amount of hydroxy groups from component C) is preferably from about 4 to about 10 %, and most preferably from about 6 to about 9%.

The polyurethane dispersions of the present invention are produced by reacting:
i) the above described prepolymer,
   D) a compound having an ionic or potentially ionic group and two groups which are reactive with isoycanate groups and
   E) an aminic or hydrazinic chain lengthening agent,
at an NCO to active hydrogen equivalent ratio of from about 3:1 to about 1.4:1, and preferably from about 2:1 to about 1.6:1.

Suitable organic diisocyanates include any organic compound (or compounds) which has two free isocyanate groups per molecule. Examples indude diisocyanates of the formula X(NCO)₂, with X representing a divalent aliphatic hydrocarbon radical having from 4 to 12 carbon atoms, a divalent cycloaliphatic hydrocarbon radical having from 6 to 15 carbon atoms, a divalent aromatic hydrocarbon radical having from 6 to 15 carbon atoms or a divalent araliphatic hydrocarbon radical having from 7 to 15 carbon atoms. Further examples of compounds that are usable as a diisocyanate component are known and are described, for example, by W. Siefken in Justus Liebig's Annalen der Chemie, 562, pp. 75-136.

Examples of diisocyanates which are preferably used include tetramethylene diisocyanate, methylpentamethylene diisocyanate, 1 ,6-hexamethylene diisocyanate, dodecamethylene diisocyanate, 1,4-diisocyanatocyclohexane, 1-isocyanato-3,3,5-trimethyl-5-isocyanatomethyl cyclohexane, 4,4'-diisocyanatobenzene, 2,4-diisocyanatotoluene, 2,6-diisocyanatotoluene, 4,4'-diisocyanatodiphenylmethane, 2,2'- and 2,4'-diisocyanatodiphenylmethane, p-xylylene diisocyanate, p-isopropylidene diisocyanate, 1,3- and 1,4-diisocyanatomethyl benzene and mixtures thereof.

1-Isocyanato-3,3,5-trimethyl-5-isocyanatomethyl cyclohexane; 1,6-hexamethylene diisocyanate; 4,4'-diisocyanatodicyclohexylmethane; 2,4- and 2,6-diisocyanatotoluene or mixtures of these isomers; 4,4'-, 2,4'- and 2,2'-diisocyanatodiphenylmethane (MDI monomers) or mixtures of these isomers, are most preferred.

It is also possible to use other isocyanates known in the polyurethane art, such as, modified isocyanates having, for example, carbodiimide groups, allophanate groups, uretdione groups, urethane groups and/or biuret groups.

Suitable dihydroxy compounds are those having two hydroxy groups and having number average molecular weights of from about 700 to about 16,000, and preferably from about 750 to about 5000. Examples include polyethers, polyesters, polycarbonates, polylactones and polyamides. Mixtures of various such compounds are also within the scope of the present invention.

The polyester diol(s) may be prepared in known manner from aliphatic, cycloaliphatic or aromatic dicarboxylic or polycarboxylic acids or anhydrides thereof (for example, succinic, glutaric, adipic, pimelic, suberic, azelaic, sebacic, nonanedicarboxylic, decanedicarboxylic, terephthalic, isophthalic, o-phthalic, tetrahydrophthalic, hexahydrophthalic or trimellitic acid) as well as acid anhydrides (such as o-phthalic, trimellitic or succinic acid anhydride or a mixture thereof) and dihydric alcohols such as, for example, ethanediol, diethylene, triethylene, tetraethylene glycol, 1,2-propanediol, dipropylene, tripropylene, tetrapropylene glycol, 1,3-propanediol, 1,4-butanediol, 1,3-butanediol, 2,3-butanediol, 1,5-pentanediol, 1,6-hexanediol, 2,2-dimethyl-1,3-propanediol, 1,4-dihydroxycyclohexane, 1,4-dimethylolcyclohexane, 1,8-octanediol, 1,10-decanediol, 1,12-dodecanediol or mixtures thereof. Cycloaliphatic and/or aromatic dihydroxyl compounds are, of course, also suitable as the dihydric alcohol(s) for the preparation of the polyester polyol(s). The corresponding polycarboxylic acid anhydrides or corresponding polycarboxylic acid esters of low alcohols, or mixtures thereof, may also be used in place of the free polycarboxylic acid for the preparation of the polyesters.

The polyester diols may naturally also be homopolymers or copolymers of lactones, which are preferably obtained by addition reactions of lactones or lactone mixtures, such as butyrolactone, ε-caprolactone and/or methyl-ε-caprolactone with the suitable difunctional starter molecules such as, for example, the low molecular weight dilyhydric alcohols mentioned above. The corresponding polymers of ε-caprolactone are preferred.

Polycarbonates having hydroxyl groups are also considered to be suitable dihydroxyl components. They may be prepared by reaction of diol(s) such as 1,4-butanediol and/or 1,6-hexanediol with diaryl carbonate(s), for example diphenyl carbonate, dialkyl or phosgene.

The polyaddition products of styrene oxides, and of ethylene oxide, propylene oxide, tetrahydrofuran, butylene oxide and epichlorohydrin, as well as co-addition and graft products thereof, as well as polyether diols obtained by condensation of dihydric alcohols or mixtures thereof and the polyether diols obtained by alkoxylation of dihydric alcohols are examples of suitable polyether diols.

Mixtures of the above-described dihydroxy compounds can also be used.

The trihydroxy component of the present invention is a triol of the formula:

R-(OH)₃

where R is a saturated straight chain or branched chain aliphatic group of from 2 to 8 carbon atoms. Specifically useful triols include trimethylol propane, trimethylol ethane, glycerol, the various isomers of butane triol, pentane triol, hexane triol, heptane triol and octane triol. The preferred triol is trimethylolpropane.

The amount of triol used is such that the hydroxy groups from the triol amount to from about 2 to about 15% based on the total amount of hydroxy equivalents used to produce the prepolymer. The amount of diisocyanate used to produce the prepolymer is such that the isocyanate content of the prepolymer is from about 1 to about 6% by weight and preferably from about 2 to about 3 % by weight.

Component D) is a compound having an ionic or potentially ionic group and two groups that are reactive with isoycanate groups. Such compounds contain two isocyanate-reactive groups and an ionic group or group capable of forming an ionic group. The ionic group or portentially ionic group can be selected from the group consisting of a ternary or quaternary ammonium groups, a groups convertible into such a group, a carboxyl group, a carboxylate group, a sulfonic acid group and a sulfonate group. The at least partial conversion of the groups convertible into salt groups of the type mentioned may take place before or during the mixing with water. Specific compounds include diols containing sulfonate groups as described in German published applications 2,446,440 and 2,437,218; diols containing carboxylate groups or carboxyl groups convertible into carboxylate groups and/or diaminosulfonates of the type described in Canadian patent 928,323, such as for example the sodium salt of N-(2-aminoethyl)-2-aminoethane sulfonic acid (AAS), dimethylol propionic acid or the sodium salt of N-(2-aminoethyl)-2- aminopropionic acid. Additional useful compounds include aminoalcohols containing tertiary amine nitrogen (wherein the tertiary nitrogen atoms may be at least partly converted into ternary or quaternary ammonium groups by neutralization or quaternization during or after the isocyanate polyaddition reaction). Specific useful compounds includeN-methyldiethanolamine, N- butyldiethanolamine, N-methyldiisopropanolamine, N-ethyldiethanolamine, N-ethyldiisopropanolamine or N,N'-bis-(2-hydroxyethyl)-perhydropyrazine.

When cationic centers are to be incorporated in the polyurethanes or NCO-prepolymers to be dispersed in accordance with the invention, it is preferably achieved using synthesis components containing tertiary amino groups of the type mentioned by way of example with subsequent conversion of the tertiary amino groups incorporated into the corresponding ammonium groups by neutralization with inorganic or organic acids (such as hydrochloric acid, acetic acid, fumaric acid, maleic acid, cyanoacetic acid, lactic acid, tartaric acid, oxalic acid, N-methyl-N-(methylaminocarbonyl)-aminomethane sulfonic acid or phosphoric acid) or by quaternization with suitable quaternizing agents such as ethylchloride, methyliodide, dimethylsulfate, benzylchloride, chloroacetic acid ethylester or bromoacetamide. Other examples of suitable neutralizing or quaternizing agents can be found in published German application 2,827,156. Basically, this neutralization or quaternization of the synthesis components containing tertiary nitrogen may be carried out before or during the isocyanate polyaddition reaction, although this is less preferred. It is also possible to introduce ternary or quaternary ammonium groups into the polyisocyanate polyaddition products through polyether polyols containing tertiary amino groups with subsequent neutralization or quaternization of the tertiary amino groups. However, this is also not preferred.

When carboxylate groups are to be incorporated in the polyurethanes or NCO-prepolymers to be dispersed in accordance with the invention, it may be done using components containing carboxylate groups, i.e. neutralized carboxyl groups, and isocyanate-reactive groups such as the triethylammonium salt of dimethylol propionic acid, or by incorporating compounds containing free carboxyl groups and isocyanate-reactive groups with subsequent neutralization of the incorporated carboxyl groups. One particularly suitable method for incorporating carboxylate groups is to use free dimethylol propionic acid in the preparation of the polyurethanes or NCO-prepolymers and subsequently neutralize the carboxyl group with a suitable neutralizing agent, for example triethylamine or sodium hydroxide.

When sulfonate groups are to be incorporated in the polyurethanes or NCO-prepolymers, it is best done by using compounds containing sulfonate groups and isocyanate-reactive groups, for example the above-mentioned aliphatic diols containing sulfonate groups according to published German applications 2,446,440 or 2,437,128.

The quantity in which the ionic or potentially ionic components are used or rather the degree of neutralization or quaternization is selected so that the polyurethanes ultimately obtained contain up to about 200, preferably about 1 to about 200, more preferably from about 2 to about 150 and most preferably about 5 to about 100 milliequivalents of ionic groups, particularly N ^{⊕},-COO^{θ} or -SO₃^{θ}, per 100 g solids.

It is also possible to incorporate both carboxylic and sulfonate groups in the polyurethanes or NCO-prepolymers to be dispersed in accordance with the invention. The simultaneous incorporation of anionic groups (carboxylate and/or sulfonate groups) and ammonium groups, in accordance with the teachings of published German application 2,721,985, is also possible in principle, but is not preferred.

Component E) is an aminic or hydrazinic chain lengthening agent, preferably containing at least two aminic or hydrazinic amino groups and having a molecular weight of from about 32 to about 400. Specifically useful compounds include diamine, hexamethylene diamine, isophorone diamine, 2,4-diaminotoluene, 4, 4'-diaminodiphenylmethane, 4,4'-diaminodicyclohexylmethane, diethylene triamine, triethylene tetramine, hydrazine and hydrazine hydrate. These compounds may also be used in blocked form, i.e. in particular in the form of the corresponding ketimines or ketazines (reaction products of amines or hydrazine with simple ketones such as acetone, methylethylketone or methylisobutylketone). When blocked chain-lengthening agents are used, the isocyanate-reactive groups are only released under the hydrolytic influence of water.

The aqueous polyurethane dispersions of the present invention are generally prepared by a process which is described by D. Dieterich in Houben-Weyl: Methoden der Organischen Chemie, Vol. E20, pp 1670-1681 (1987). The so-called "acetone process" is preferred. In this process the aqueous dispersions are synthesized in a multi-stage process.

In the first stage (following the prepolymer preparation), the prepolymer according to the invention is dissolved in an organic, at least partially water-miscible solvent having no isocyanate-reactive groups. The preferred solvent is acetone. However, other solvents such as, for example, 2-butanone, tetrahydrofuran, dioxane, N-methylformamide, N-methylacetamide or N-methylpyrrolidone may be used, either as such or included in small amounts. The quantities are chosen such that a solids content of from about 20 to about 80% by weight, preferably from about 30 to about 50% by weight, results.

The prepolymer solution is then reacted with the mixture(s) of the anionic group (or potentially anioinic group) containing compound and chain lengthening compound, preferably dissolved in one of the aforementioned solvents or in water at an NCO to active hydrogen equivalent ratio of from about 3:1 to about 1.4:1, and preferably from about 2:1 to about 1.6:1, to obtain the high molecular weight polyurethane resin. The quantities of components are such that the polyurethanes ultimately obtained contain up to about 200, preferably about 1 to 200, more preferably 2 to 150 and most preferably about 5 to 100 milliequivalents of ionic groups, particularly N ^{⊕},-COO^{θ} or -SO₃^{θ}, per 100 g solids.

When a component having a free carboxylic acid group or sulfonic acid group is used, the acid groups are neutralized with a neutralizing agent before the addition of the water necessary for dispersing, at a ratio of from about 50 to about 100 equivalent %, in relation to free acid groups.

The high molecular weight polyurethane resin is precipitated in the form of a finely divided dispersion by the addition of water to the solution. The organic solvent may optionally be distilled off in whole or in part at reduced pressure. The quantity of water is such that the aqueous dispersions comprise from about 30 to about 60% by weight, preferably from about 35 to about 50% by weight, solids.

The dispersions may be processed by conventional processes (e.g., by spraying or by the dip process or coagulation process) to obtain films, foils, surface coatings, coatings, finishes and for impregnation of the most widely varied substrates. If a spray process is used, the dispersion is sprayed onto a substrate and dried to evaporate the water present. If the dip or coagulation process is used, a coagulating agent is first applied to a substrate (or mold form), the dispersion is then applied to the substrate, the wet coating is treated with warm water to remove excess coagulating agent, then the coating is heated to dry it and the resulting film is removed. The dispersions are particularly suitable for the production of films and for the manufacture of polyurethane gloves and condoms by the dip process or coagulation process.

The polyurethane dispersions may also, depending on their intended use, contain conventional auxiliary agents and additives, such as, for example, plasticizers, pigments, defoaming agents, soft-feel additives or fillers.

It is also possible to combine the aqueous dispersions of the invention, with other dispersions such as, for example, polyacrylate dispersions, natural and synthetic rubber latices such as, for example, NBR (nitrile-butadiene rubber), chloroprene or other homopolymers and copolymers such as, for example, ethyl vinyl acetate or ethyl vinyl alcohol. In fact, the benefit of the present invention are also obtained when the dispersions of the present invention are combined with aqueous polyurethane dispersions that are prepared from prepolymers which do not include the triols as presently used. In such combined dispersions, the dispersion of the present invention is present in an amount of from about 70 to about 95% by weight, and preferably from about 80 to about 90% by weight, based on the total weight of the combined dispersions.

The materials produced from the polyurethane dispersions of the present invention have satisfactory solvent resistance while at the same time have good minimum tear strength and minimum ultimate elongation.

The invention is further illustrated but is not intended to be limited by the following examples in which all parts and percentages are by weight, unless otherwise specified.

### EXAMPLES

### Example 1

The following materials were charged to a reactor equipped with heating mantle, stirrer, nitrogen inlet, reflux condenser and charging funnel: 385.5 grams of a polyesterdiol (OH No. 66; adipic acid hexanediol neopentyl glycol ester) and 1.52 grams of trimethylolpropane (TMP). The stirrer was then turned on and the mixture was heated to 70°C. At that temperature 70.1 grams of hexamethylene diisocyanate (Desmodur® H, Bayer Corp.) were added. The reaction mixture was allowed to exotherm to 75°C and was then held at 75°C until the isocyanate content reached 2.98% by weight (theory = 3.30%). The mixture was diluted with 815 grams acetone and cooled to 41.5°C. A solution of 3.84 grams ethylene-diamine and 9.95 grams of the sodium salt of ethylene-diamine-2-ethane-sulfonic-acid (AAS salt) in 140 grams distilled water were added within 30 seconds. Fifteen minutes later 710 grams of distilled water were added and the acetone was subsequently distilled off under reduced pressure.

A fine particle dispersion having a particle size of the dispersed phase of about 155 nm and a solids content of 38% by weight was obtained.

### Example 2

The following materials were charged to a reactor equipped with heating mantle, stirrer, nitrogen inlet, reflux condenser and charging funnel: 377.0 grams of a polyesterdiol (OH No. 66; adipic acid hexanediol neopentyl glycol ester) and 2.99 grams of trimethylolpropane (TMP). The stirrer was then turned on and the mixture was heated to 70°C. At that temperature 70.5 grams of hexamethylene diisocyanate (Desmodur® H, Bayer Corp.) were added. The reaction mixture was allowed to exotherm to 70 to 75°C and was then held at 75°C until the isocyanate content reached 2.94% by weight (theory = 3.30%). The mixture was diluted with 815 grams acetone and cooled to 41.5°C. A solution of 3.84 grams ethylene-diamine and 9.95 grams of the sodium salt of ethylene-diamin-2-ethan-sulfonic-acid (AAS salt) in 140 grams distilled water were added within 30 seconds. 15 minutes later 710 grams of distilled water were added and the acetone was distilled off under reduced pressure subsequently.

A fine particle dispersion having a particle size of the dispersed phase of about 135 nm and a solid content of 38% by weight was obtained.

### Comparative Example A - No triol

The following materials were charged to a reactor equipped with heating mantle, stirrer, nitrogen inlet, reflux condenser and charging funnel: 1133.0 grams of a polyesterdiol (OH No. 66; adipic acid hexanediol neopentyl glycol ester). The stirrer was then turned on and the mixture was heated to 70°C. At that temperature 201 grams of hexamethylene diisocyanate -(Desmodur® H, Bayer Corp.) were added. The reaction mixture was allowed to exotherm to 85°C and was then held at 85°C until the isocyanate content reached 2.84% by weight (theory = 3.32%). The mixture was diluted with 2405 grams acetone and cooled to 47°C. A solution of 11.5 grams ethylene-diamine, and 53.3 grams of the sodium salt of ethylene-diamin-2-ethan-sulfonic-acid (AAS salt) in 300 grams distilled water were added within 30 seconds. Fifteen minutes later 1800 grams of distilled water were added and the acetone was distilled off under reduced pressure subsequently.

A fine particle dispersion having a particle size of the dispersed phase of approx. 87 nm and a solid content of 40% by weight was obtained.

### Comparative Example B - Use of triamine instead of triol;

The following materials were charged to a reactor equipped with heating mantle, stirrer, nitrogen inlet, reflux condenser and charging funnel: 380.0 grams of a polyesterdiol (OH No. 66; adipic acid hexanediol neopentyl glycol ester). The stirrer was then turned on and the mixture was heated to 70°C. At that temperature 67.2 grams of hexamethylene diisocyanate (Desmodur® H, Bayer Corp.) were added. The reaction mixture was allowed to exotherm to 70 to 75°C and was then held at 75°C till the isocyanate content reached 2.98% by weight (theory = 3.32%). The mixture was diluted with 795 grams acetone and cooled to 41.5°C. A solution of 3.49 grams ethylene-diamine, 0.38 grams diethylenetriamine and 8.05 grams of the sodium salt of ethylene-diamine-2-ethan-sulfonic-acid (AAS salt) in 100 grams distilled water were added within 30 seconds. 15 minutes later 610 grams of distilled water were added and the acetone was distilled off under reduced pressure subsequently.

A fine particle dispersion having a solid content of 41 % by weight was obtained.

### Preparation and Testing of film - Examples 1 and 2

The coagulant solution consisted of a mixture of calcium carbonate and calcium nitrate. The coagulant solution was heated to 140°F and continuously stirred. A porcelain tube was preheated to 150°F. The tube was dipped into the coagulant solution and withdrawn slowly. The tube was rotated to evenly distribute the coagulant. The tube was allowed to air dry for 60 seconds. The tube was then dipped into the polyurethane dispersion and withdrawn slowly. The tube was rotated to evenly distribute the dispersion. The coating was allowed to air dry for 60 seconds. The coated tube was dipped into a container of 120°F water for 2 minutes. The tube was placed in a 300°F oven for 8 minutes. The cured film was dusted with corn starch and removed from the tube by rolling the film down. A flat film was obtained by cutting the polyurethane tube down one side. A dumbbell shaped specimen was cut from the polyurethane film. The ends of the dumbbell were stretched so that the center portion of the film was lengthened by 100%, i.e., a one inch portion is stretched to two inches. One drop of Sterillium solution (a disinfectant in an isopropanol solution) was deposited on the middle of the stretched section of the film. The amount of swelling or the breaking of the film was as set forth in table 1.

**Table 1**

| Resin Sample | Test Result from Isopropanol Test |
|---|---|
| Example 1 | Film swelled one inch |
| Example 2 | Film swelled one quarter inch |
| Comparative Example A | Film broke |
| Comparative Example B | Film broke |

### Example 3

90 parts by weight of the dispersion of Example 1 was blended with 10 parts by weight of the dispersion of Comparative example A.

### Example 4

80 parts by weight of the dispersion of Example 1 was blended with 20 parts by weight of the dispersion of Comparative example A.

### Comparative Example C

90 parts by weight of the dispersion of Example 1 was belnded with 10 parts by weight of Santicizer 160 plasticizer (butyl benzyl phthalate).

### Preparation and Testing of film - Examples 3 and 4

The films were prepared in the same manner as prepared in Examples 1 and 2. The filmswere tested in same way, with the goal being the ability to pass a two-inch stretch. In addition, tensile testing was conducted according to ASTM D412. Goals were to have % elongation at break of about 600, psi at 100% modulus of about 300 and psi at ultimate break of >3000.

The results were as set forth in Table 2

**Table 2**

| Resin Sample | Test Result from Isopropanol Test | Elongtion % | Modulus at 100% Elongation psi | Tensile Strength at Break psi |
|---|---|---|---|---|
| Example 1 | Passed two inch stretch | 560 | 400 | 3600 |
| Example A | Failed two inch stretch | 650 | 200 | 1600 |
| Example 3 | Passed two inch stretch | 620 | 320 | 3250 |
| Example 4 | Passed two inch stretch | 630 | 300 | 2950 |
| Example C | Failed two inch stretch | 580 | 310 | 2500 |

### Example 5

The following materials were charged to a reactor equipped with heating mantle, stirrer, nitrogen inlet, reflux condenser and charging funnel: 268.01 grams of a polyesterdiol (OH No. 66; adipic acid hexanediol neopentyl glycol ester), 2.4 grams of trimethylolpropane (TMP), and 53.44 grams of 1,6-hexane diol orthophtalate (Stepanol PH 56 - a polyester diol having an OH number of 56). The stirrer was then turned on and the mixture was heated to 70°C. At that temperature 61.75 grams of hexamethylene diisocyanate (Desmodur®, Bayer Corp.) were added. The reaction mixture was allowed to exotherm to 75°C and was then held at 70°C until the isocyanate content reached 3.23% by weight (theory = 3.41 %). The mixture was diluted with 675 grams acetone and cooled to 43.5°C. A solution of 2.27 grams ethylene-diamine and 17.6grams of the sodium salt of ethylene-diamine-2-ethanesulfonic-acid (AAS salt) in 120 grams distilled water were added within 5 minutes. Five minutes later 595 grams of distilled water were added and the acetone was subsequently distilled off under reduced pressure.

A fine particle dispersion having a particle size of the dispersed phase of about 184 nm and a solid content of 38% by weight was obtained.

Films were produced as in Examples 1 through 4, with tensile testing was conducted according to ASTM D0412. The films both passed the two inch stretch test

Results were as set forth in Table 3.

**Table 3**

| Formulation | Stress at 100% (psi) | Stress at 200% (psi) | Stress at 300% (psi) | Stress at 500% (psi) | Stress at max. load (psi) | % Strain at max. load |
|---|---|---|---|---|---|---|
| Target | Less than 300 | | | | Above 2500 | Above 650 |
| Example 3 | 320 | 401.5 | 506.9 | 1012.8 | 2868.1 | 626.2 |
| Example 5 | 263.8 | 335.6 | 440.0 | 786.6 | 2470.3 | 720.7 |

Since manufacturing of gloves calls for continuing agitation of the dispersion, simulation of the process was conducted. Samples of the dispersions were slowly stirred for a week at room temperature. After that viscosity and particle size were compared to the initial. Typically, if a dispersion doesn't have shear stability, viscosity and particle size drastically increase. The results were as set forth in Table 4.

**Table 4**

| Composition | Initial average particle size, micron | Final average particle size (after mix), micron | Initial viscosity @25°C, cps | Final viscosity after mix @25°C, cps |
|---|---|---|---|---|
| Example 3 | 0.220 | 8.32 | 120 | 1100 |
| Example 5 | 0.154 | 6.4 | 207 | 960 |

Although the invention has been described in detail in the foregoing for the purpose of illustration, it is to be understood that such detail is solely for that purpose and that variations can be made therein by those skilled in the art without departing from the spirit and scope of the invention except as it may be limited by the claims.

## Claims

1. A prepolymer having an NCO content of from about 1 to about 6% by weight, and being prepared by reacting:
A) an organic diisocyanate,
B) at least one dihydroxy compound having a number average molecular weight of from about 700 to about 16,000, and
C) a trihydroxy component of the formula:
R-(OH)₃
where R is a saturated straight chain or branched chain
aliphatic group of from 2 to 8 carbon atoms, and
wherein the amount of component C) is such that the hydroxy groups from component C) amount to from about 2 to about 15 % based on the total amount of hydroxy equivalents used to produce the prepolymer.

2. The prepolymer of Claim 1, wherein the amount of component C) is such that the hydroxy groups from component C) amount to from about 4 to about 10 % based on the total amount of hydroxy equivalents used to produce the prepolymer.

3. The prepolymer of Claim 3, wherein the amount of component C) is such that the hydroxy groups from component C) amount to from about 6 to about 9 % based on the total amount of hydroxy equivalents used to produce the prepolymer.

4. The prepolymer of Claim 1, wherein said trihydroxy compound is selected from the group consisting of trimethylol propane, trimethylol ethane, glycerol, the various isomers of butane triol, pentane triol, hexane triol, heptane triol and octane triol.

5. The prepolymer of Claim 4, wherein said trihydroxy compound is trimethylol propane.

6. The prepolymer of Claim 1, having an NCO content of from about 2 to about 3% by weight

7. An aqueous polyurethane dispersion produced by reacting:
i) the prepolymer of Claim 1,
D) a compound having an ionic or potentially ionic group and two groups which are reactive with isoycanate groups and
E) an aminic or hydrazinic chain lengthening agent,
at an NCO to active hydrogen equivalent ratio of from about 3:1 to about 1.4:1.

8. The aqueous dispersion of Claim 7, wherein the NCO to active hydrogen equivalent ratio of from about 2:1 to about 1.6:1.

9. The aqueous dispersion of Claim 7, wherein the amount of component D) is such that the degree of neutralization or quaternization in the dispersion is from about 1 to about 200 milliequivalents of ionic groups per 100 g solids.

10. The aqueous dispersion of Claim 7 comprises from about 30 to about 60% by weight solids.

11. An aqueous dispersion comprising from about 70 to about 95% by weight of the dispersion of Claim 2 and from about 5 to about 30% by weight of an aqueous polyurethane dispersion produced from a prepolymer that does not use a trihydroxy compound in its preparation.

12. In a process for forming a film by spraying an aqueous dispersion onto a substrate and drying the dispersion to evaporate water present, the improvement wherein the dispersion is the aqueous polyurethane dispersion of Claim 2.

13. In a process for preparing a film by a process comprising coating a substrate with a coagulating agent, applying a dispersion to the substrate so-coated, treating the so-coated substrate with warm water to remove excess coagulating agent, heating the resultant coating to dry it and removing the resultant film from the substrate, the improvement wherein the dispersion is the aqueous polyurethane dispersion of Claim 7.

14. A film produced according to the process of Claim 13.

15. A medical glove produced according to the process of Claim 13.
